# EUROPEAN PATENT APPLICATION

(11) **EP 1 811 024 A1**
(43) Date of publication of application: **25.07.2007**
(21) Application number: 05790513.5
(22) Date of filing: 04.10.2005
(51) Int. Cl.: C12N 15/09, A61K 31/713, A61P 1/16, A61P 31/14, A61P 35/00

(54) **OLIGO DOUBLE-STRANDED RNA AND MEDICINAL COMPOSITION**

(30) Priority: 05.10.2004 JP 2004292041
(71) Applicant: Nippon Shinyaku Co., Ltd., Kyoto-shi, Kyoto 601-8550 (JP)
(72) Inventor: HIRABAYASHI, Kazuko, 307, Green Palace Kaikonoyashiro, Kyoto-shi, Kyoto 6168105 (JP); NAITO, Haruna, Kyoto-shi, Kyoto 6020036 (JP)
(74) Representative: Vossius & Partner
(86) International application number: PCT/JP2005/018331
(87) International publication number: WO 2006/038608

(57) **Abstract**

It is intended to provide an double-stranded RNA oligonucleotide inhibiting the replication of HCV and a medicinal composition containing this double-stranded RNA oligonucleotide for treating and/or preventing a disease induced by infection with HCV. The present invention relates to a double-stranded RNA oligonucleotide inhibiting the replication of HCV which is **characterized by** having a double-stranded portion comprising a pair of RNAs, SEQ ID NO.3 and SEQ ID NO.4 without the two deoxythymidine monophosphate (hereinafter referred to as "dT") nucleotides of at the respective 3'-termini, or a pair of RNAs, SEQ ID NO.2 and SEQ ID NO.5, without the two dT nucleotides at the respective 3'-terminals; and a medicinal composition containing this double-stranded RNA oligonucleotide together with a suitable carrier.

## Description

### Technical Field

The present invention relates to a double-stranded RNA oligonucleotide and a pharmaceutical composition comprising the double-stranded RNA oligonucleotide.

### Background Art

Hepatitis C Virus (hereinafter referred to as "HCV") is classified into genotypes 1a, 1b, 2a, 2b, 3a and 3b. When all genotypes are considered together, the number of HCV-infected patients worldwide is about 170 million. In Japan, the number of HCV-infected patients is said to be about 1.5-2.0 million. About 80% of HCV infections become chronic and progress through chronic hepatitis to cirrhosis and hepatoma in about 20-30 years. In Japan, about 80% of all HCV-infected patients are infected with HCV genotype 1b and have a large quantity of virus in the serum. In addition, there are 34,000 hepatoma-related deaths per year, and about 75% of these cases of hepatoma are caused by HCV infection.

For HCV infection, an antiviral therapy mainly with IFN is provided. It has proved to be effective in reducing the incidence of hepatoma and in prolonging life. However, in IFN therapy, the complete response rate (that is, the proportion of patients whose serum HCV RNA has been negative for more than 24 weeks after completion of therapy) for genotype 1b HCV patients with a high virus titer, which account for the majority of Japanese patients, is only about 16% of all cases, so that the effect is limited. Therefore, a next-generation HCV remedy to follow IFN is expected. Review articles on HCV infection and methods of treatment may be referred to (see, for example, "Progress in Medicine", 2003, vol.4, pp.1109-1131).

When a double-stranded RNA (hereinafter referred to as "dsRNA") is introduced into a cell, an RNA complementary to the dsRNA is specifically degraded, and the synthesis of the gene product encoded by the RNA is thereby inhibited. This phenomenon is called RNA interference (hereinafter referred to as "RNAi").
In addition, a short double-stranded RNA, which has an activity inhibiting the expression of a protein by RNAi, is referred to as short interfering RNA (hereinafter referred to as "siRNA"). RNAi techniques using siRNA have been reviewed (see, for example, "Protein, Nucleic Acid and Enzyme", 2002, vol.47, pp.1939-1945 and "Cell", 2002, vol.110, no.5, pp.563-574).

Methods for introducing siRNA into a cell include a method using as a vehicle a carrier such as a cationic liposomes or other cationic carriers, and a method of direct introduction into a cell such as calcium phosphate method, electroporation or microinjection. In addition, a method in which an expression vector incorporating with a siRNA-coding sequence is introduced into a cell so as to express siRNA in the cell has also been investigated (see, for example, "Nature Biotechnology", 2002, vol.20, no.5, pp.497-500 and "Science", 2002, vol.296, pp.550-553).

In recent years, there have been some reports concerning siRNA targeting HCV RNA. However, none of them have shown satisfactory effects in the treatment of Hepatitis C (see, for example, International Publication WO03/070750, International Publication WO02/081494, "Proceedings of the National Academy of Sciences", 2003, vol.100, no.1, pp.235-240 and "Proceedings of the National Academy of Sciences", 2003, vol.100, no.5, pp.2783-2788).

### Disclosure of Invention

The main object of the present invention is to provide a double-stranded RNA oligonucleotide to inhibit HCV replication and a pharmaceutical composition comprising the double-stranded RNA oligonucleotide for the treatment and/or prevention of diseases which are caused by HCV infection.

The present inventors have conducted extensive researches to find that a double-stranded RNA oligonucleotide comprising as a double-stranded portion a pair of RNAs, SEQ ID NO.3 and SEQ ID NO.4, without the two deoxythymidine monophosphate (hereinafter referred to as "dT") nucleotides at the respective 3'-termini or a pair of RNAs, SEQ ID NO.2 and SEQ ID NO.5, without the two dT nucleotides at the respective 3'-termini can inhibit HCV replication and achieve the object stated above.

The present invention includes, for example:
(1) A double-stranded RNA oligonucleotide comprising as a double-stranded portion a pair of RNAs, SEQ ID NO.3 and SEQ ID NO.4, without the two dT nucleotides at the respective 3'-termini or a pair of RNAs, SEQ ID NO.2 and SEQ ID NO.5, without the two dT nucleotides at the respective 3'-termini. The double-stranded RNA oligonucleotide is hereinafter referred to as "the present double-stranded RNA oligonucleotide".
(2) An RNA having a sequence selected from SEQ ID NO.2, SEQ ID NO.3 and SEQ ID NO.5 without the two dT nucleotides at the respective 3'-termini and retaining inhibitory activity against HCV replication when constituting a double-stranded RNA oligonucleotide with the complementary RNA thereof. The RNA is hereinafter referred to as "the present RNA".
(3) A DNA having the same sequence as the present RNA of (2) above wherein the sugar part of ribonucleotide is replaced with D-2-deoxyribose and uracil (U) in the nucleotide sequence is replaced with thymine (T). The DNA is hereinafter referred to as "the present DNA".
(4) An expression vector for RNA, wherein the present DNA described in (3) above is inserted.
(5) A pharmaceutical composition comprising the present double-stranded RNA oligonucleotide of (1) above and a carrier. The pharmaceutical composition is hereinafter referred to as "the present composition.
(6) A pharmaceutical composition comprising the expression vector DNA described in (4) above and a carrier.

### Best Mode for Carrying Out the Invention

### I. The present double-stranded RNA oligonucleotide

The present double-stranded RNA oligonucleotide may include, for example, a double-stranded RNA oligonucleotide comprising as a double-stranded portion a pair of RNAs, SEQ ID NO.3 and SEQ ID NO.4, without the two dT nucleotides at the respective 3'-termini or a pair of RNAs, SEQ ID NO:2 and SEQ ID NO.5, without the two dT nucleotides at the respective 3'-termini.

The two RNAs consisting of the nucleotide sequences SEQ ID NO.1 and SEQ ID NO.2 without the two dT nucleotides at the respective 3'-termini are respectively sense RNAs homologous to a part of the internal ribosome entry site (IRES) in the HCV RNA registered in GenBank under accession number AB080299. The two RNAs consisting of the nucleotide sequences SEQ ID NO.4 and SEQ ID NO.5 without the two dT nucleotides at the respective 3'-termini are respectively antisense RNAs complementary to SEQ ID NO.1 and SEQ ID NO.2 without the two dT nucleotides at the respective 3'-termini. The RNA consisting of the nucleotide sequence SEQ ID NO.3 without the two dT nucleotides at the 3'-terminus is the same as the RNA consisting of the nucleotide sequence SEQ ID NO.1 without the two dT nucleotides at the 3'-terminus, except that the C nucleotide at the 3'-terminus is changed to an A nucleotide.

The "double-stranded portion" means the portion in the double-stranded RNA oligonucleotide in which a pair of nucleic acids constituting the double-stranded RNA oligonucleotide forms a double strand, which contains a sequence homologous to HCV RNA. In this connection, when there is a single strand at the 3'- and/or 5'-terminal side following the double-stranded portion in the respective RNA strands, this is called an overhang.

"Inhibitory activity against HCV replication" means activity in which the present double-stranded RNA oligonucleotide, when introduced into a cell, can inhibit HCV replication in comparison with the case where the double-stranded RNA oligonucleotide is not present. The "inhibitory activity against HCV replication is provided" specifically means the case where the degree of inhibition(%) is more than 50%, preferably, more than 75% and more preferably, more than 90%. When the quantity of HCV RNA in the cells transfected with the present double-stranded RNA oligonucleotides is equal to that of a negative control, the degree of inhibition is regarded as 0%.

So long as the present double-stranded RNA oligonucleotide retains inhibitory activity against HCV replication, it may contain one or more (for example, 4 or fewer) deleted, substituted, inserted or added nucleotides in either or both sequences of a double-stranded portion.

In addition, provided that the present double-stranded RNA oligonucleotide retains inhibitory activity against HCV replication, one or more of the ribonucleotides in either or both RNAs of the double-stranded portion may be replaced by one or more deoxyribonucleotides or modified.
As used herein, "modified deoxyribonucleotide" means a deoxyribonucleotide which is modified in at least a part of its structure, such as the deoxyribose or phosphate back bone, in order to enhance in vivo stability, for example by increasing nuclease resistance. Examples of the modification may include a modification of the 2'-position of the deoxyribose, a modification of positions other than 2'-position of the deoxyribose, and a modification of the phosphate backbone. The modification of 2'-position of deoxyribose may include replacement of 2'-hydrogen of deoxyribose with OR, R, R', OR, SH, SR, NH₂, NHR, NR₂, N₃, CN, F, Cl, Br, I, etc., wherein "R" represents alkyl or aryl.
Preferable examples of the "alkyl" R may include straight or branched alkyls having 1 to 6 carbon atoms. More particularly, it may include methyl, ethyl, n-propyl, isopropyl, n-butyl, isobutyl, sec-butyl, tert-butyl, n-pentyl, isopentyl, neopentyl, tert-pentyl, n-hexyl and isohexyl, etc. The "alkyl" may be substituted. For example, it can be substituted by 1 to 3 substituents which can be selected from the group consisting of halogen, alkyl, alkoxy, cyano and nitro. Examples of the "halogen" may include fluorine, chlorine, bromine and iodine. The "alkyl" may include the same groups described above. The "alkoxy" may include straight or branched alkoxy groups having 1 to 6 carbons, for example, methoxy, ethoxy, n-propoxy, isopropoxy, n-butoxy, isobutoxy, sec-butoxy, tert-butoxy, n-pentyloxy, isopentyloxy, n-hexyloxy and isohexyloxy. Particularly, an alkoxy having 1 to 3 carbon atoms is preferable.
The "aryl" R is preferably an aryl having 6 to 10 carbon atoms. Specifically, it may include phenyl, alpha-naphthyl and beta-naphthyl. Particularly, phenyl is preferable.
In addition, R' represents alkylene. Examples of the "alkylene" R' are straight or branched alkylene having 1 to 6 carbon atoms. Specifically, it may include methylene, ethylene, trimethylene, tetramethylene, pentamethylene, hexamethylene, 2-(ethyl) trimethylene, 1-(methyl) tetramethylene.
The modifications of the other parts of the deoxyribose may include a 4'-thio derivatives. The modifications of the phosphate backbone may include a phosphorothioate derivative, a phosphorodithioate derivative, an alkylphosphonate derivative, a phosphoroamidate derivative, and the like.

In addition, the present double-stranded RNA oligonucleotide may contain 1 to 4 nucleotide nucleotide as an overhang at the 3'- or 5'-terminus of at least one of the RNA strands in the double-stranded portion, provided that it retains inhibitory activity against HCV replication. When an overhang exists, the nucleotide(s) constituting the overhang may be either ribonucleotide or deoxyribonucleotide.
Preferably, the present double-stranded RNA oligonucleotide has two nucleotides as an overhang. More preferably, the present double-stranded RNA oligonucleotide has as an overhang two dT nucleotides or two uridine monophosphates (hereinafter referred to as "U") at the 3'-terminus of the sense strand and/or the 3'-terminus of the antisense strand of the present double-stranded RNA oligonucleotide, two nucleotides of HCV RNA, which are next to 3'-terminus of a part of HCV RNA identical to the sense strand of the present double-stranded RNA oligonucleotide at the 3'-terminus of the sense strand, or two nucleotides complementary to two nucleotides of HCV RNA, which are next to 5'-terminus of a part of HCV RNA complementary to the antisense strand of the present double-stranded RNA oligonucleotide at the 3'-terminal side of the antisense RNA.

Further, so long as the present double-stranded RNA oligonucleotide retains inhibitory activity against HCV replication, it may be modified at least partially in the riboses or phosphate backbone in order to enhance in vivo stability for example by increasing nuclease resistance. Examples of the modification may include a modification of the 2'-position of the ribose, a modification of positions other than 2'-position of the ribose and a modification of the phosphate backbone. The modification of 2'-position of a ribose includes replacement of 2'-hydroxy group of the ribose with OR, R, R', OR, SH, SR, NH₂, NHR, NR₂, N₃, CN, F, Cl, Br, I, etc., wherein "R" represents alkyl or aryl.
Preferable examples of the "alkyl" R may include straight or branched alkyls having 1 to 6 carbon atoms. Particularly, it may include methyl, ethyl, n- propyl, isopropyl, n- butyl, isobutyl, sec-butyl, tert-butyl, n-pentyl, isopentyl, neopentyl, tert-pentyl, n-hexyl and isohexyl, etc. The "alkyl" may be substituted by 1 to 3 substituents which can be selected from the group consisting of halogen, alkyl, alkoxy, cyano and nitro and the like. Examples of the "halogen" may include fluorine, chlorine, bromine and iodine. The "alkyl" may include the same alkyl described above. The "alkoxy" includes straight or branched alkoxy groups having 1 to 6 carbon atoms such as methoxy, ethoxy, n-propoxy, isopropoxy, n-butoxy, isobutoxy, sec-butoxy, tert-butoxy, n-pentyloxy, isopentyloxy, n-hexyloxy and isohexyloxy. Particularly, an alkoxy having 1 to 3 carbon atom(s) is preferable.
The "aryl" R is preferably aryl having 6 to 10 carbon atoms. Specifically, it may include phenyl, alpha-naphthyl and beta-naphthyl. Particularly, phenyl is preferable.
In addition, R' represents alkylene. Examples of the "alkylene" R' are straight or branched alkylenes having 1 to 6 carbon atoms. Specifically, it may include methylene, ethylene, trimethylene, tetramethylene, pentamethylene, hexamethylene, 2-(ethyl) trimethylene and 1-(methyl) tetramethylene.
The modifications of the other parts of the ribose include a 4'-thio derivative. The modifications of the phosphate backbone include a phosphorothioate derivative, a phosphorodithioate derivative, an alkylphosphonate derivative, a phosphoroamidate derivative, and the like.

The present double-stranded RNA oligonucleotide can be prepared by mixing and annealing 1 molar equivalent of one RNA with the other RNA of 0.5 to 2 molar equivalents, preferably of 0.9 to 1.1 molar equivalents, and more preferably of an equimolar amount. Annealing can be carried out by methods well known to persons skilled in the art. For example, annealing can be carried out by mixing two selected RNAs, heating the mixture at about 94°C for about 5 minutes, and then gradually cooling it down to room temperature. In addition, a mixture of two selected RNAs may be used directly as the present double-stranded RNA oligonucleotide without the annealing step.

The present double-stranded RNA oligonucleotide may be introduced into a cell using a carrier described below. The present double-stranded RNA oligonucleotide may also be introduced directly into a cell by a calcium phosphate method, electroporation or microinjection, or the like.

### II. The present RNA and the present DNA

The present RNA may include an RNA consisting of a sequence selected from SEQ ID NO.2, SEQ ID NO.3 and SEQ ID NO.5 without the two dT nucleotides at the 3'-termini, and retaining inhibitory activity against the HCV replication when constituting an double-stranded RNA oligonucleotide with a complementary RNA thereof.

The present RNA may contain one or more (for example, 4 or fewer) deleted, substited, inserted or added nucleotides.

As in the case of the present double-stranded RNA oligonucleotide described above, the present RNA may be substituted by a deoxyribonucleotide(s) or a modified deoxyribonucleotide(s) in a part of ribonucleotides in the present RNA.

The present RNA may contain 1 to 4 additional nucleotide(s) at the 3'- or 5'-terminus. The said nucleotide may be either ribonucleotide or deoxyribonucleotide. Preferably, the present RNA has two of the said nucleotides. More preferably, the present RNA has as the said nucleotides two dT nucleotides or two U nucleotides, two nucleotides of HCV RNA, which are next to the 3'-terminus of a part of HCV RNA identical to the present RNA at the 3'-terminus of the present RNA when the present RNA is a sense strand, or two nucleotides complementary to two nucleotides of HCV RNA, which are next to the 5'-terminus of a part of the HCV RNA complementary to the present RNA at the 3'-terminus of the present RNA when the present RNA is an antisense RNA.

As in the case of the present double-stranded RNA oligonucleotide described above, the present RNA may be modified at least partially in the riboses or phosphate backbone in order to enhance in vivo stability for example by increasing nuclease resistance.

The example of the present DNA may include a DNA having the same sequence as the present RNA wherein the sugar part of ribonucleotide is replaced with D-2-deoxyribose and uracil (U) in the nucleotide sequence is replaced with thymine (T).

The present DNA may be incorporated into a plasmid to express the present double-stranded RNA oligonucleotide, or may be used as a template DNA to obtain the present double-stranded RNA oligonucleotide by in vitro transcription. Also, it may be used as an antisense probe.

The present RNA and the present DNA can be synthesized in the solid or the liquid phase by the phosphoramidite method or triester method as is well known to persons skilled in the art. The most conventional method is solid phase synthesis by the phosphoramidite method. It is possible to perform the synthesis by using a nucleic acid automatic synthesizer or manually.
After termination of the synthesis on the solid phase, the desired product is released from the solid phase and purified after removal of the protecting group. The purity of the present RNA or the present DNA finally obtained may suitably be 90% or more, and preferably 95% or more.

### III. The present composition

The present composition may include a pharmaceutical composition comprising the present double-stranded RNA oligonucleotide and a carrier. The said carrier means a carrier which is effective in introducing the present double-stranded RNA oligonucleotide into a cell. The present composition can be used for treating and/or preventing diseases caused by HCV infection. Diseases caused by HCV infection include, for example, chronic hepatitis, cirrhosis and hepatoma.

The carrier related to the present composition is not particularly limited, but a cationic carrier such as a cationic liposome or a cationic polymer, or a carrier utilizing a virus envelope, which is pharmaceutically acceptable can be used. The cationic liposome may include, for example, a liposome essentially consisting of 2-O-(2-diethylaminoethyl)carbamoyl-1,3-O-dioleoylglycerol and a phospholipid (hereinafter referred to as "liposome A"), Oligofectamine (RTM; Invitrogen), Lipofectin (RTM; Invitrogen), Lipofectamine (RTM; Invitrogen), Lipofectamine 2000 (RTM; Invitrogen), DMRIE-C (RTM; Invitrogen), Gene Silencer (RTM; Gene Therapy Systems), Trans Messenger (RTM; QIAGEN), TransIT TKO (RTM; Mirus), or the like. Among them, liposome A is preferable. The preferable cationic polymer may include Jet SI (RTM; Qbiogene) and Jet-PEI (RTM; polyethylenimine, Qbiogene). The preferable carrier utilizing a virus envelope may include Genome One (RTM; HVJ-E liposome, Ishihara Sangyo) and the like.

The concentration of the present double-stranded RNA oligonucleotide is suitably in the range from 0.1 nM to 100 µM, preferably 1 nM to 10 µM, more preferably 10 nM to 1 µM, although it depends on the kinds of the carrier, etc.
In addition, the weight ratio of the carrier to the present double-stranded RNA oligonucleotide contained in the present composition (carrier/present double-stranded RNA oligonucleotide) may depend on the properties of the present double-stranded RNA oligonucleotide, the kind of the carrier, etc, but the ratio may suitably be in the range from 0.1 to 100 parts by weight, preferably in the range from 1 to 50 parts by weight, more preferably in the range from 10 to 20 parts by weight.

The present composition may contain one or more pharmaceutically acceptable additives in addition to the present double-stranded RNA oligonucleotide and a carrier described above. The additives may include emulsifying auxiliary agents such as aliphatic acids having 6 to 22 carbon atoms and pharmaceutically acceptable salts thereof, albumin and dextran; stabilizers such as cholesterol and phosphatidic acid; the isotonic agents such as sodium chloride, glucose, maltose, lactose, sucrose, and trehalose; pH adjusters such as hydrochloric acid, sulfuric acid, phosphoric acid, acetic acid, sodium hydroxide, potassium hydroxide, triethanolamine and the like. These can be used alone or as a mixture of two or more thereof. The content of the additives in the present compositions may suitably be 90% by weight or less, preferably 70% by weight or less, or more preferably 50% by weight or less.

The present composition can be prepared by adding the present double-stranded RNA oligonucleotide to a liquid dispersion of a carrier and stirring it appropriately. In addition, it is possible to add the additive at a suitable step before or after the addition of the present double-stranded RNA oligonucleotide.
The aqueous solutions used for adding the present double-stranded RNA oligonucleotide are not particularly limited so long as they are pharmaceutically acceptable and may include, for example, water for injection, distilled water for injection, an electrolytic solution such as saline, a glucose solution and a maltose solution. In addition, further conditions such as pH and temperature may suitably be determined by persons skilled in the art.

The present composition can be prepared in the form of liquid formulations or in the form of freeze-dried preparations. The freeze-dried preparations can be prepared by freeze-drying the present composition in the form of liquid formulations by conventional methods. For example, after sterilizing the present composition in the form of liquid formulations, the composition is dispensed into vials in predetermined amounts, and then the composition is subjected to preliminary freezing at about -40°C to -20°C for about 2 hours, followed by a first drying at about 0°C to 10°C under reduced pressure and a secondary drying at about 15°C to 25°C under reduced pressure. Thereafter, in general, the vials are filled with nitrogen gas and sealed with stoppers to give lyophilized preparations of the present composition.

For use, the freeze-dried preparation of the present composition is ordinarily reconstituted by adding thereto an appropriate solution (reconstituting solution). The reconstituting solutions may include water for injection, saline, and the other standard infusions. The amount of the reconstituting solution may vary depending on the usage and the like, and is not particularly limited, but an appropriate amount may be 0.5 to 2 times the amount of the original liquid formulation before the freeze-drying or not more than 500 mL.

The composition of the present invention may preferably be administered with a dose unit form, and can be administered by intravenous administration, intra-arterial administration, oral administration, intra-tissue administration, transdermal administration, transmucosal administration, or rectal administration to animals including humans. Particularly, intravenous administration, transdermal administration, and transmucosal administration are preferred. The composition is administered in formulations appropriate for the above-mentioned types of administration, such as various injections, an oral preparation, drops, an inhalant, eye drops, an ointment, a lotion, and a suppository.

It is desirable to determine the dosage of the present composition in consideration of the kind of the present double-stranded RNA oligonucleotide included in the composition, formulation, subject's condition such as age and body weight, route of administration, nature and degree of disease, or the like, and usually the dosage is in the range from 0.1 mg to 10 g/day/subject as the present double-stranded RNA oligonucleotide for an adult, preferably in the range from 1 mg to 500 mg/day/subject. This dose may vary depending also on the type of target disease, the administration method, and the target molecule. Therefore, the dose may be lower than that or may need to be increased in some cases. The composition may be administered once or several times a day and at intervals of 1 to several days.

Another embodiment of the present composition includes a pharmaceutical composition comprising an expression vector for RNA, wherein the present DNA is incorporated, and a carrier. The expression vector may comprise some of the present DNAs or well-known DNAs. Especially, for example, the expression vector may incorporate DNA which can express both a sense strand and an antisense strand constituting the present double-stranded RNA oligonucleotide. In the same manner as the present composition comprising the present double-stranded RNA oligonucleotide, additives can be added to the present composition.
The concentration of the expression vector included in the present composition is suitably in the range from 0.1nM to 100µM, preferably 1nM to 10µM, more preferably 10nM to 1µM, although it depends on the kinds of the carrier etc. The weight ratio of the carrier to the expression vector in the present composition (carrier/expression vector) may vary depending on the properties of the expression vector, the kind of the carrier, etc, but, the ratio may appropriately be in the range from 0.1 to 100 parts by weight, preferably in the range from 1 to 50 parts by weight, more preferably in the range from 10 to 20 parts by weight. In addition, the content of a carrier contained in the said composition is similar to the case of the present composition comprising the present double-stranded RNA oligonucleotide, and the method of preparation etc. is also similar to the case of the present composition.
Furthermore, it is desirable to determine the method and route of administration to suit the pathology of the subject in a similar manner to the case of the present composition, and the dosage may also be determined in consideration of the drug, formulation, subject's condition such as age and body weight, route of administration, and nature and degree of disease.

### Example

The present invention is illustrated more specifically below by referring to the following Example and Test Example. However, the present invention is not limited thereto.

### Example Preparation of a pharmaceutical composition

### (1) Synthesis of an RNA constituting a double-stranded RNA oligonucleotide

The synthesis of RNAs constituting an double-stranded RNA oligonucleotide was outsourced to Japan Bioservice (Saitama Pref., Japan).

### (2) Preparation of double-stranded RNA oligonucleotide #130

An aqueous solution of the double-stranded RNA oligonucleotide #130 was prepared in the following manner. The present RNAs, synthesized as described in (1) above mentioned, consisting of the nucleotide sequences SEQ ID No.1 and SEQ ID No.4 were respectively dissolved in water for injection so as to be 120 µM, and then 16.6 µl each were mixed in a test tube, followed by the addition of 966.8 µl water for injection.

### (3) Preparation of double-stranded RNA oligonucleotide #130A

An aqueous solution of the double-stranded RNA oligonucleotide #130A was prepared as described in (2) above, except that the present RNAs SEQ ID NO.3 and SEQ ID NO.4 synthesized as described in (1) above were used.

### (4) Preparation of double-stranded RNA oligonucleotide #258

The aqueous solution of the double-stranded RNA oligonucleotide #258 was prepared as described in (2) above, except that the present RNAs SEQ ID NO.2 and SEQ ID NO.5 synthesized as described in (1) above were used.

### (5) Preparation of a pharmaceutical composition

A liposome A-dispersed solution of 16 mg/ml, which comprises as essential components 6 parts by weight of 2-O-(2-diethylaminoethyl) carbamoyl-1,3-O-dioleoylglycerol and 10 parts by weight of purified egg yolk lecithin was prepared as a carrier by a known method (WO94/19314).
One ml of a liposome A-dispersed solution was prepared by adding 973.4 µl of water for injection to 26.6 µl of the above mentioned liposome A-dispersed solution. One milliliter of the above double-stranded RNA oligonucleotide solution prepared as described in (2) to (4) above was slowly added to 1 ml of the liposome A-dispersed solution with stirring. The particles of the complex were homogenized by dispersion in a 600 W bath-type ultrasonicator for 2 minutes. By the above procedure, a pharmaceutical composition with a double-stranded RNA oligonucleotide in which the final concentration of double-stranded RNA oligonucleotide was 1µM was prepared, assuming that duplex formation in the present RNAs is complete.

### Test Example Screening of double-stranded RNA oligonucleotide

### (1) Cell Culture

In the screening of the following (2), HuH-7#50-1, which is a human hepatoma-derived cell line, was used. Details of HuH-7#50-1 are described in Biochemical and Biophysical Research Communications, 2000, vol. 293, pp.993-999.
As a cell culture medium at the time of screening, DMEM medium (Nissui Co., Japan) containing 10% FBS (Sankojunyaku Co., Japan) was used. Except when the screening was performed, 400 µg/ml of G418 (GibcoBRL Co.) was also included in the cell culture medium described above.

### (2) Methods of screening and evaluation thereof

In each petri dish having a diameter of 6 cm, 3 × 10⁵ cells were seeded and cultured overnight at 37°C, under an atmosphere of 5% CO₂ in air. The next day, the medium was changed by removing the medium from the dish by aspiration and then adding 2.7 ml of fresh culture medium.
The pharmaceutical composition prepared in "Example" (0.3 ml) was added to give a final volume of 3 ml. At this point, the final concentration of the present double-stranded RNA oligonucleotide was 100 nM. Incubation was then performed in a CO₂ incubator for 96 hours. The cells were washed twice with PBS, harvested with a cell scraper, and total RNA was isolated by a well-known method. Then, using a 2.5 µg aliquot of the total RNA as a template, a cDNA was synthesized by reverse transcription using a ThermoScript reverse transcriptase polymerase chain reaction (RT-PCR) system (Invitrogen Corporation).
Using the cDNA contained in the reverse transcription reaction mixture as a template in the PCR reaction, PCR amplification specific for HCV RNA and a constitutively expressed gene GAPDH (D-glyceraldehyde-3-phosphate dehydrogenase) was conducted using a real-time PCR detection/quantification system (ABI Prism 7000), and the amount of RNA was determined semiquantitatively. In PCR amplification of HCV RNA, 100 ng of cDNA derived from total RNA was used as a template, and in PCR amplification of GAPDH, 0.5 ng of cDNA derived from total RNA was used as a template. As a negative control, cDNA derived from total RNA isolated from cells to which distilled water had been added was used.
The amount of HCV RNA was calculated as a relative ratio when the amount of> GADPH mRNA was set to 1. The result was evaluated as degree of inhibition, which was regarded as 0% when the quantity of HCV RNA in the cell added with the pharmaceutical composition is equal to the quantity of HCV RNA in negative control.

### (3) Test result

The results of the screening described above are shown in Table 1. Table 1 shows that the two double-stranded RNA oligonucleotides, #130A (SEQ ID NO.3 and SEQ ID NO.4) and #258 (SEQ ID NO.2 and SEQ ID NO.5) had a marked inhibitory activity against HCV replication. In addition, the double-stranded RNA oligonucleotide #130A (SEQ ID NO.3 and SEQ ID NO.4) showed a much higher inhibitory activity against HCV replication compared to that #130 (SEQ ID NO.1 and SEQ ID NO. 4).

**Table 1**

| double-stranded RNA oligonucleotide | Sequence (5' → 3') | Degree of inhibition (%) |
|---|---|---|
| 130 | CGGGAGAGCCAUAGUGGUC-dTdT (SEQ ID N0.1) | 63 |
| | GACCACUAUGGCUCUCCCG-dTdT (SEQ ID NO.4) | |
| 130A | CGGGAGAGCCAUAGUGGUA-dTdT (SEQ ID NO.3) | 93 |
| | GACCACUAUGGCUCUCCCG-dTdT (SEQ ID NO.4) | |
| | GUAGUGUUGGGUCGCGAAA-dTdT (SEQ ID NO.2) | 91 |
| 258 | UUUCGCGACCCAACACUAC-dTdT (SEQ ID NO.5) | |

## Claims

1. A double-stranded RNA oligonucleotide comprising as a double-stranded portion a pair of RNAs, SEQ ID NO.3 and SEQ ID NO.4, without two deoxythymidine monophosphate nucleotides at the respective 3'-termini or a pair of RNAs, SEQ ID NO.2 and SEQ ID NO.5, without two deoxythymidine monophosphate nucleotides at the respective 3'-termini.

2. The double-stranded RNA oligonucleotide according to Claim 1, wherein one or more deleted, substituted, inserted or added nucleotides are contained in at least one of the RNA strands of a double-stranded portion, said double-stranded RNA oligonucleotide retaining an replication inhibitory activity against Hepatitis C Virus.

3. The double-stranded RNA oligonucleotide according to Claim 1, wherein a part or all of ribonucleotides constituting at least one of the RNA strands in the double-stranded portion are replaced with deoxyribonucleotides or modified deoxyribonucleotides, said double-stranded RNA oligonucleotide retaining inhibitory activity against Hepatitis C Virus replication.

4. The double-stranded RNA oligonucleotide according to Claim 1, which has an addition of a nucleotide of 1- to 4-nucleotide as an overhang at the 3'-terminus of at least one of the RNA strands or at the 5'-terminus of at least one of the RNA strands in the double-stranded portion, and retains inhibitory activity against Hepatitis C Virus replication.

5. The double-stranded RNA oligonucleotide according to Claim 4, wherein the 1- to 4-nucleotide added to the 3'-terminus or 5'-terminus as an overhang is a deoxyribonucleotide.

6. The double-stranded RNA oligonucleotide according to Claim 4, wherein two deoxythymidine monophosphate (dT) nucleotides are added as an overhang to the 3'-terminus of at least one of the RNA strands in the double-stranded portion.

7. The double-stranded RNA oligonucleotide according to Claim 1, wherein riboses or phosphate backbones of a part of nucleotides constituting a double-stranded portion are modified, said double-stranded RNA oligonucleotide retaining inhibitory activity against Hepatitis C Virus replication.

8. The double-stranded RNA oligonucleotide according to Claim 7, wherein the modification of riboses or phosphate backbones comprises one or more modifications selected from a modification of a 2'-hydroxyl group of the ribose by replacement with a group selected from OR, R, R'OR, SH, SR, NH₂, NHR, NR₂, N₃, CN, F, Cl, Br, or I, wherein R represents alkyl or aryl, and R' represents alkylene; a modification of the ribose to a 4'-thio derivative; and a modification of the phosphate backbone to a phosphorothioate, a phosphorodithioate, an alkylphosphonate, or a phosphoroamidate.

9. An RNA comprising SEQ ID NO.2, SEQ ID NO.3 or SEQ ID NO.5 without the two deoxythymidine monophosphate (dT) nucleotides at the 3'-termini, said RNA retaining inhibitory activity against Hepatitis C Virus replication when constituting an double-stranded RNA oligonucleotide with a complementary RNA thereof.

10. The RNA according to Claim 9, wherein one or more deleted, substituted, inserted, or added nucleotides are contained in the nucleotide sequence.

11. The RNA according to Claim 9, wherein a part of ribonucleotides constituting the RNA is replaced with deoxyribonucleotides or modified deoxyribonucleotides.

12. The RNA according to Claim 9, which has an addition of a nucleotide of 1- to 4-nucleotide at the 3'-terminus or the 5'-terminus of the strands.

13. The RNA according to Claim 12, wherein the 1- to 4-nucleotide nucleotide added to the 3'-terminus or the 5'-terminus is a deoxyribonucleotide.

14. The RNA according to Claim 12, which has an addition of two nucleotides at the 3'-terminus, and the nucleotides added are deoxythymidine monophosphate.

15. The RNA according to Claim 9, wherein a part or all of riboses or a phosphate backbone constituting the nucleotides in RNA strand are modified.

16. A DNA having the same sequence as the RNA according to any one of Claims 9 to 15, wherein the sugar part of ribonucleotide is replaced with D-2-deoxyribose and uracil (U) in the nucleotide sequence is replaced with thymine (T).

17. An RNA expression vector, wherein the DNA according to Claim 16 is inserted.

18. A pharmaceutical composition which comprises the double-stranded RNA oligonucleotide according to any one of Claims 1 to 8 and a carrier.

19. A pharmaceutical composition which comprises the expression vector according to Claim 17 and a carrier.

20. The pharmaceutical composition according to Claim 18 or 19, wherein the carrier is a cationic carrier.

21. The pharmaceutical composition according to Claim 20, wherein the cationic carrier is a liposome essentially comprising 2-O-(2-diethylaminoethyl) carbamoyl-1,3-O-dioleoylglycerol and a phospholipid.

22. The pharmaceutical composition according to claim 21, wherein the phospholipid is lecithin.

23. The pharmaceutical composition according to Claim 18 or 19, which is used for treating and/or preventing a disease for which replication inhibition of Hepatitis C Virus is desired.

24. The pharmaceutical composition according to Claim 21, wherein the disease for which replication inhibition of Hepatitis C Virus is desired is chronic hepatitis, cirrhosis or hepatoma.


SEQUENCE LISTING

<110> Nippon Shinyaku Co., LTD.

<120> Oligo dsRNA which inhibits a replication of Hepatitis C virus, and
pharmaceutical composition comprising thereof.

<130> B-354PCT

<160> 6

<210> 1
<211> 21
<212> DNA
<213> Artificial sequence

<220>
<223> synthesized DNA/RNA; a strand of 130, wherein 2 nucleotides of 3'-terminus
are constructed with DNA, and the rest are RNA.

<400> 1
cgggagagcc auagugguct t                                                    21

<210> 2
<211> 21
<212> DNA
<213> Artificial sequence

<220>
<223> synthesized DNA/RNA; a strand of 258, wherein 2 nucleotides of 3'-terminus
are constructed with DNA, and the rest are RNA.

<400> 2
guaguguugg gucgcgaaat t                                                    21

<210> 3
<211> 21
<212> DNA
<213> Artificial sequence

<220>
<223> synthesized DNA/RNA; a strand of 130A, wherein 2 nucleotides of
3'-terminus are constructed with DNA, and the rest are RNA.

<400> 3
cgggagagcc auagugguat t                                                    21

<210> 4
<211> 21
<212> DNA
<213> Artificial sequence

<220>
<223> synthesized DNA/RNA; a strand of 130 and 130A, wherein 2 nucleotides of
3'-terminus are constructed with DNA, and the rest are RNA.

<400> 4
gaccacuaug gcucucccgt t                                                    21

<210> 5
<211> 21
<212> DNA
<213> Artificial sequence

<220>
<223> synthesized DNA/RNA; a strand of 258, wherein 2 nucleotides of 3'-terminus
are constructed with DNA, and the rest are RNA.

<400> 5
uuucgcgacc caacacuact t                                                21

<210> 6
<211> 9587
<212> DNA
<213> Hepatitic C virus

<220>
<221> source
<222> 1..9587
<223> /organism="Hepatitis C virus"
      /mol_type="genomic RNA"
      /isolate="M1LE"
      /db_xref="taxon:11103"
      /note="genotype 1b"

<220>
<221> 5'UTR
<222> 1..341

<220>
<221> CDS
<222> 342..9374
<223> /product="polyprotein"
      /protein_id="BAC54896.1"
      /db_xref="GI:27544244"
      /translation="MSTNPKPQRKTKRNTNRRPQDVKFPGGGQIVGGVYLLPRRGPRL
      GVRATRKTSERSQPRGRRQPIPKARRPEGRTWAQPGYPWPLYGDEGMGWAGWLLSPRG
      SRPNWGPTDPRRRSRNLGKVIDTLTCGFADLMGYIPLVGAPLGGVARALAHGVRVLED
      GVNYATGNLPGCSFSIFLLALLSCLTTPASAYEVRNVSGIYHVTNDCSNSSIVYEAAD
      IIMHTPGCVPCVREKNTSRCWVALTPTLAARNISVPTTTIRRHVDLLVGTAAFCSAMY
      VGDLCGSVFLVSQLFTFSPRRHETVQDCNCSLYPGHVSGHRMAWDMMMNWSPTAALVV
      SQLLRIPQAIVDMVAGAHWGVLAGLAYYSMVGNWAKVLIVMLLFAGADGTTHVTGGVQ
      AHGAYGLASLFNVGPHQKIQLVNTNGSWHINRTALNCNDTLQTGFLAALFYKHRFNAS
      GCPERMASCRPIDKFAQGWGPITYAEPDRLDQRPYCWHYPPRPCGIVPALEVCGPVYC
      FTPSPVVVGTTDRFGVPTYSWGENETDVLLLNNTRPPQGNWFGCTWMNNTGFTKTCGG
      PPCNIGGVGNNTLTCPTDCFRKHPEATYTKCGSGPWLTPRCLVDYPYRLWHYPCTVNF
      TVFKVRMYVGGIEHRLDAACNWTRGERCDLEDRDRSELSPLLLSTTEWQILPCSFTTL
      PALSTGLIHLHRNIVDVQYLYGIGSAVVSFAIKWEYILLLFLLLADARVCACLWMMLL
      IAQAEAALENLVVLNAASVAGAHGILSFLVFFCAAWCIKGKLVPGAAYAFYGVWPLLL
      LLLALPPRAYAMDREMAASCGGAVFVGLAFLTLSPYYKVFLARLIWWLQYLITRAEAH
      LQMWVPPLNIRGGRDAIILLMCAVHPELIFDITKLLLAILGPLMVLQAGITRVPYFVR
      AQGLIRACMLVRKAAGGHYVQMAFVKLAALTGTYVYDHLAPLQHWAHTGLRDLAVAVE
      PVVFSDMETKIITWGADTAACGDIILGLPVSARRGREILLGPADSLEGQGWRLLAPIT
      AYSQQTRGLLGCIITSLTGRDKNQVEGEVQVVSTATQSFLATCINGVCWTVFHGAGSK
      TLAGPKGPITQMYTNVDQDLVGWQAPPGARSMTPCTCGSSDLYLVTRHADVIPVRRRG
      DGRGSLLSPRPVSYLKGSSGGPLLCPSGHAVGIFRAAVCTRGVAKAVDFIPVESMETT
      MRSPVFTDNSSPPAVPQTFQVAHLHAPTGSGKSTKVPAAYAAQGYKVLVLNPSVAATL
      GFGAYMSKAYGTDPNIRTGVRTITTGAPITYSTYGKFLADGGCSGGAYDIIICDECHS
      TDSTTILGIGTVLDQAETAGARLVVLATATPPGSVTVPHPNIEEVALSNTGEIPFYGK
      AIPIEVIKGGRHLIFCHSKKKCDELAAKLSSLGLNAVAYYRGLDVSVIPTSGDVVVVA
      TDALMTGFTGDFDSVIDCNTCVTQTVDFSLDPTFTIETTTVPQDAVSRSQRRGRTGRG
      RGGIYRFVTPGERPSGMFDSSVLCECYDAGCAWYELTPAETSIRLRAYLNTPGLPVCQ
      DHLEFWESVFTGLTHIDAHFLSQTKQAGDNFPYLVAYQATVCARAQAPPPSWDQMWKC
      LTRLKPTLHGPTPLLYRLGAVQNEVTLTHPITKYIMACMSADLEVVTSTWVLVGGVLA
      ALAAYCLTTGSVVIVGRIILSGKPAVIPDREVLYREFDEMEECASHLPYIEQGMQLAE
      QFKQKALGLLQTATKQAEAAAPVVESKWRALETFWAKHMWNFISGIQYLAGLSTLPGN
      PAIASLMAFTASITSPLTTQSTLLFNILGGWVAAQLAPPSAASAFVGAGVAGAAVGSI
      GLGKVLVDILAGYGAGVAGALVAFKIMSGEMPSTEDLVNLLPAILSPGALVVGVVCAA
      ILRRHVGPGEGAVQWMNRLIAFASRGNHVSPTHYVPESDAARVTQILSSLTITQLLK
      RLHQWINEDCSTPCSGSWLRDIWDWICTVLADFKTWLKSKLMPQLPGVPFFSCQRGYK
      GVWRGDGIMQTTCPCGAQITGHVKNGSMRIAGPKTCSNTWHGTFPINAYTTGPCTPSP
      APNYSRALWRVAAEEYVEVTRVGDFHYVTGMTTDNVKCPCQVPAPEFFTEVDGVQLHR
      YAPACKPLLRDEVTFQVGLNQYLVGSQLPCEPEPDVAVLTSMLTDPSHITAETAKRRL
      ARGSPPSLASSSASQLSAPSLKATCTTHHDSPDADLIEANLLWRQEMGGNITRVESEN
      KVVILDSFDPLRAEEDEREVSVAAEILRKSRKFPPALPVWARPDYNPPLLEPWKDPDY
      VPPVVHGCPLPPTKAPPIPPPRRKRTVALTESTVSSALAELATKTFGSSGSSAADSGT

ATAPPDQASDDGDKGSDVESYSSMPPLEGEPGDPDLSDGSWSTVSEEASEDVVCCSMS
YSWTGALITPCAAEESKLPINPLSNSLLRHHNMVYATTSRSAGLRQKKVTFDRLQVLD
DHYRDVLKEMKAKASTVKAKLLSIEEACKLTPPHSAKSKYGYGAKDVRNLSSRAVNHI
RSVWEDLLEDTETPIDTTIMAKSEVFCVQPEKGGRKPARLIVFPDLGVRVCEKMALYD
VVSTLPQAVMGSSYGFQYSPKQRVEFLVNTWKSKKCPMGFSYDTRCFDSTVTENDIRV
EESIYQCCDLAPEAKLAIKSLTERLYVGGPLTNSKGQNCGYRRCRASGVLTTSCGNTL
TCYLKASAACRAAKLQDCTMLVNGDDLVVICESAGTQEDAASLRVFTEAMTRYSAPPG
DPPQPEYDLELITSCSSNVSVAHDASGKRVYYLTRDPTTPLARAAWETARHTPVNSWL
GNIIMYAPTLWARMILMTHFFSILLAQEQLEKALDCQIYGACYSIEPLDLPQIIERLH
GLSAFSLHSYSPGEINRVASCLRKLGVPPLRVWRHRARSVRAKLLSQGGRAATCGKYL
FNWAVKTKLKLTPIPAASRLDLSGWFVAGYSGGDIYHSLSRARPRWFMWCLLLLSVGV
GIYLLPNR"

<400> 6
gccagccccc gattgggggc gacactccac catagatcac tcccctgtga ggaactactg 60
tcttcacgca gaaagcgtct agccatggcg ttagtatgag tgtcgtgcag cctccaggac 120
cccccctccc gggagagcca tagtggtctg cggaaccggt gagtacaccg gaattgccag 180
gacgaccggg tcctttcttg gattaacccg ctcaatgcct ggagatttgg gcgtgccccc 240
gcaagactgc tagccgagta gtgttgggtc gcgaaaggcc ttgtggtact gcctgatagg 300
gtgcttgcga gtgccccggg aggtctcgta gaccgtgcac catgagcacg aatcctaaac 360
ctcaaagaaa aaccaaacgt aacaccaacc gccgcccaca ggacgttaag ttcccgggcg 420
gtggtcagat cgttggtgga gtttacctgt tgccgcgcag gggccccagg ttgggtgtgc 480
gcgcgactag gaagacttcc gagcggtcgc aacctcgtgg aaggcgacaa cctatcccca 540
aggctcgccg gcccgagggc aggacctggg cccagcccgg gtacccttgg cccctctatg 600
gtgacgaggg catggggtgg gcaggatggc tcctgtcacc ccgtggctct cggcctaatt 660
ggggccccac agacccccgg cgtaggtcgc gtaatttggg taaggtcatc gataccctca 720
catgcggctt cgccgacctc atggggtaca ttccgcttgt cggcgcccct ctaggaggcg 780
ttgccagggc cctggcgcat ggcgtccggg ttctggagga cggcgtgaat tatgcaacag 840
ggaatttgcc cggttgctct ttctctatct tcctcttggc tctgctgtct tgcttgacta 900
ccccagcttc cgcttatgaa gtgcgcaacg tgtccgggat ataccatgtc acgaacgact 960
gctccaactc aagcattgtg tatgaggcag cggacatcat catgcatacc cccgggtgcg 1020
tgccctgcgt tcgggagaag aacacctccc gttgctgggt agcgctcact cccacgctcg 1080
ggacggctgc tttctgctct gctatgtacg tgggggatct ctgcggatct gttttcctcg 1140
tctcccagct gttcaccttc tcacctcgcc ggcatgaaac agtacaggac tgcaattgct 1200
cactctatcc tggccacgta tcaggtcacc gtatggcttg ggacatgatg atgaactggt 1260
cgcctacagc agctctagtg gtgtcgcagt tgctccggat cccacaagct atcgtggata 1320
tggtggcggg ggcccactgg ggagtcctgg cgggccttgc ctactattcc atggtgggga 1380
actgggccaa ggttttgatt gtgatgctgc tctttgccgg cgctgacggg accacccacg 1440
tgacaggggg ggtgcaagcc cacggtgctt acgggcttgc atccctcttt aacgttgggc 1500
cccatcagaa aatccagctt gtaaatacaa atggcagctg gcacatcaac aggactgcct 1560
taaactgcaa tgacaccctc cagaccggat tccttgccgc gctgttctac aaacacaggt 1620
tcaacgcgtc cggatgccca gagcgtatgg ccagctgccg tcccattgac aagttcgctc 1680
agggatgggg tcccatcact tatgctgagc ctgacaggct ggaccagagg ccttattgct 1740
ggcactaccc accccgaccg tgtggtatcg tacccgcgtt ggaggtgtgt ggtccagtgt 1800
attgcttcac ccccaagccct gttgtggtgg ggacgaccga tcgtttcggc gtccctacct 1860
atagctgggg ggagaatgag acggatgtgc tgctcctcaa caacacgcgg cctccgcaag 1920
gcaattggtt cggctgtacg tggatgaata acaccgggtt caccaagacg tgtgggggcc 1980
ctccgtgtaa catcgggggg gtcggcaaca acaccttgac ctgccccacg gattgcttcc 2040
ggaagcaccc cgaggctact tacacaaaat gtggttcagg gccttggttg acacctaggt 2100
gcttagttga ctacccctat aggctctggc actacccctg cactgtcaac tttaccgtct 2160
tcaaggtcag gatgtatgtg gggggcattg agcacaggct cgatgctgca tgcaattgga 2220
ctcgaggaga gcgctgtgac ttggaggaca gggatagatc agaactcagc ccgctgttgc 2280
tgtctacaac agagtggcag atactgccct gttccttcac caccctaccg gccctgtcca 2340
ctggtttaat ccatctccac cggaacatcg tggacgtgca gtatctgtac ggcataggat 2400
cagcggttgt ctcctttgca atcaaatggg aatacatcct gttgcttttc cttctcctgg 2460
cggacgcgcg cgtctgtgcc tgcttgtgga tgatgctgct gatagcccag gctgaggccg 2520
ccttagagaa cttggtggtc ctcaatgcgg cgtccgtggc cggagcgcat ggtatcctct 2580
ccttccttgt gttcttctgc gccgcctggt gtattaaggg taagctggtc cctggggcgg 2640
cgtatgcttt ctatggcgta tggccgctgc ttctgctcct gttggcgttg ccgccacgtg 2700
cttacgccat ggaccgggag atggctgcat catgtggagg cgcagttttt gtaggattgg 2760
cattcttgac tttgtcgcca tactataaag tgttcctcgc taggctcata tggtggttac 2820
aataccttat caccagagcc gaggcgcact tgcagatgtg ggtccctcct cttaatatcc 2880
ggggggggccg cgatgccatc atcctcctca tgtgcgcggt ccacccagag ctaatctttg 2940
acatcaccaa actcctgctt gccatacttg gtccgctcat ggtgctccag gccggcataa 3000
ctagagtgcc gtacttcgta cgcgctcaag ggctcattcg tgcatgcatg ttggtgcgga 3060
aagccgccgg gggtcattat gtccaaatgg ccttcgtgaa gctggccgca ctgacaggca 3120
cgtacgttta tgaccatctt gccccactgc aacattgggc ccacacgggc ctacgagacc 3180
ttgcggtggc agtagagccc gttgtcttct ctgacatgga aaccaagatc atcacctggg 3240

gggcagatac cgcggcatgt ggggacatca tcttgggtct acccgtctcc gcccggaggg 3360
gaagggagat actcctggga ccagccgata gtcttgaggg gcaggggtgg cgactcctcg 3420
cgcccatcac ggcttactcc caacagacgc ggggcctact cggttgcatc atcaccagcc 3480
tcacaggtcg ggacaagaac caggttgagg gggaggttca agtggtttcc accgctacac 3540
aatctttcct ggcgacctgc atcaatggcg tgtgttggac tgttttccac ggtgccggct 3600
caaagaccct agccggccca aaaggtccaa tcacccaaat gtacaccaat gtagaccagg 3660
acctcgtcgg ctggcaggcg cccccggggg cacgttccat gacaccatgc acctgcggca 3720
gctcggacct ttacttggtc acgagacatg ctgatgtcat cccggtgcgc cgacggggcg 3780
acggtagggg gagccttctc tcccccaggc ccgtctccta cttgaagggc tcttcgggtg 3840
gtccgctgct ctgcccttcg gggcatgccg tgggcatctt ccgggccgct gtgtgcaccc 3900
gggggtcgc gaaggcggtg gacttcatac ccgtcgagtc tatggaaacc actatgcggt 3960
ctccagtctt cacagacaac tcatcccccc cggctgtacc gcagacattt caagtggctc 4020
atctgcacgc ccccactggc agcggcaaga gcactaaagt gccggctgca tatgcagccc 4080
aagggtacaa ggtacttgtc ctgaacccgt ccgttgctgc caccttaggt tttggggcgt 4140
atatgtctaa ggcatatggt accgacccca acatcagaac tggggtaagg accatcacca 4200
cgggcgctcc catcacgtat tccacctatg gcaagttcct cgccgacggt ggttgctccg 4260
ggggtgccta cgacatcata atatgtgatg agtgccactc aactgactcg actaccatct 4320
tgggcatcgg cacagtcctg gatcaagcgg agacggctgg agcgcggctc gtcgtgctcg 4380
ccaccgctac gcctccagga tcggtcaccg tgccgcaccc caatatcgag gaggtggccc 4440
tgtccaacac tggagagatc cccttctatg gcaaagccat ccccatcgag gtcatcaaag 4500
gggaaggca tctcatttc tgccactcca agaaaaagtg cgacgagctc gccgcaaagc 4560
tgtcgtccct cggactcaat gctgtagcgt attaccgggg tcttgatgtg tccgtcatac 4620
cgactagcgg agatgtcgtt gtcgtggcaa cggacgctct aatgacgggc tttaccggcg 4680
acttcgactc agtgatcgac tgtaatacat gtgtcactca gacagtcgac ttcagcttgg 4740
atcccacctt caccattgaa acgacgaccg tgccccaaga cgcggtatcg cgctcacagc 4800
ggcgaggtag gactggcagg ggtaggggag gcatctacag gtttgtgact ccaggggaac 4860
ggccctcggg catgttcgat tcctcggtcc tgtgtgagtg ctatgacgcg ggctgtgcgt 4920
ggtacgagct cacgcccgcc gagacctcga ttaggttgcg ggcgtaccta aatacaccag 4980
ggttgcccgt ctgccaggac catctggagt tctgggagag tgtcttcaca ggcctcaccc 5040
atatagatgc ccacttcttg tcccagacta agcaggcagg agacaacttc ccctacctgg 5100
tggcatacca ggctacagtg tgcgccaggg ctcaggctcc acctccatcg tgggatcaaa 5160
tgtggaagtg tctcacacgg ctaaaaccta cactgcacgg gccaacaccc ttgctgtata 5220
ggctaggagc cgtccaaaat gaggtcaccc tcacacaccc cataaccaaa tacatcatgg 5280
catgcatgtc ggctgacctg gaggtcgtca ccagcacttg ggtgctggta ggcggagtcc 5340
ttgcagctct ggccgcgtat tgcctgacaa caggcagcgt agtcattgtg ggcaggatca 5400
tcttgtccgg gaagccggcc gtcattcccg acagggaagt tctttaccgg gagttcgatg 5460
agatggaaga gtgcgcctca cacctccctt acatcgaaca gggaatgcag ctcgccgaac 5520
ctcccgtggt ggagtccaag tggcgcgctc ttgagacctt ctgggcgaag cacatgtgga 5640
atttcatcag cgggatacag tatttagcag gcttatccac tctgcctggg aaccccgcga 5700
tagcatcact gatggcattt acagcctcta tcaccagccc gctcaccacc caaagtaccc 5760
tcctgtttaa catcttgggg ggatgggtgg ctgcccaact cgctcccccc agcgctgctt 5820
ctgcttttgt gggcgccggc gttgccggtg cggctgttgg cagcataggc ctcgggaagg 5880
tgcttgtgga catcctggcg ggctatgggg caggggtggc tggcgcactc gtagctttta 5940
agatcatgag cggcgagatg ccctccaccg aggacctggt caatttactt cctgccatcc 6000
tctctcctgg tgccctggtt gtcggggtcg tgtgcgcagc aatactgcgc cggcacgtgg 6060
gcccaggaga gggggctgtg cagtggatga accggctgat agcgttcgct tctcgggta 6120
accacgtctc ccccacgcac tatgtgcctg agagcgacgc cgcagcgcgt gttactcaga 6180
tcctctccag ccttaccatc actcagctgc tgaagagact tcaccagtgg atcaatgagg 6240
actgttccac gccatgctcc ggctcgtggc taagggatat ttgggactgg atatgcacgg 6300
tattggctga ctttaagacc tggctcaagt ccaagctcat gccgcaatta ccgggagtcc 6360
ctttcttctc ttgccaacgc ggctacaagg gagtctggcg gggggacggt atcatgcaga 6420
ccacctgccc atgtggagca cagatcaccg gacatgtcaa gaacggttcc atgaggatcg 6480
ctgggcctaa aacctgcagc aacacgtggc atggaacatt ccccatcaac gcgtacacca 6540
cgggcccctg cacaccctcc ccggcgccaa actattccag ggcgctgtgg cgggtggctg 6600
ctgaggagta cgtggaggtt acgcgggtgg gggatttcca ctacgtgacg ggcatgacca 6660
ctgacaacgt gaagtgtcca tgccaggttc cggcccccga attcttcacg gaagtggatg 6720
gagtgcagtt gcacaggtac gctccggcgt gtaaacctct cctacgggat gaggtcacat 6780
tccaggtcgg gctcaaccaa tacctggttg ggtcgcagct cccatgcgag cccgaaccgg 6840
atgtagcagt gctcacttcc atgctcaccg atccttccca cattacagca gagacggcca 6900
agcgtaggtt ggccaggggg tctcccccct ccctggccag ctcttcagct agccagttgt 6960
ctgcgccttc cttgaaggct acatgtacta cccaccatga ctccccggac gctgacctca 7020
tcgaggccaa cctcttgtgg cggcaagaga tgggcgggaa catcacccgc gtggagtcag 7080
agaacaaggt agtaattctg gactctttcg acccgcttcg agcggaggag gatgagaggg 7140
aggtatccgt tgcggcggag atcctgcgga aatccaggaa gttcccccct gcgctgcccg 7200
tatgggcacg cccggattat aatcctccac tgctagagcc ctggaaggac ccggactatg 7260
ttcctccggt ggtacacggg tgcccattgc cgcctaccaa ggcaccccca ataccacccc 7320
cacggagaaa gaggacggtt gccctgacag agtccaccgt gtcttctgcc ttggcggagc 7380
tcgctactaa gaccttcggc agctccggat cgtcggccgc cgacagcggc acggcgactg 7440

cccctcctga ccaggcctcc gacgacggcg acaagggatc cgacgttgag tcatactcct 7500
ccatgccccc ccttgaggga gagccggggg accctgatct tagcgacggg tcttggtcta 7560
ccgtgagcga ggaggctagt gaggacgtcg tctgctgctc aatgtcctac tcatggacag 7620
gtgccctgat cacgccatgc gctgcggagg aaagcaagct gcccatcaac ccgttgagca 7680
actccctgct gcgtcaccac aatatggtct acgccacaac atcccgtagc gcaggcctgc 7740
ggcagaagaa ggtcaccttt gacaggctgc aagtcctgga cgatcattac cgggacgtgc 7800
tcaaggagat gaaggcgaag gcgtccacag ttaaggctaa gcttctatct atagaagaag 7860
cctgcaaact gacgccccca cactcggcca aatccaaata tggctatggg gcaaaggacg 7920
tccggaacct atccagcagg gccgtcaacc acatccgctc cgtgtgggag gacttgctgg 7980
aagacactga gacaccaatt gacactacca tcatggcaaa gagtgaggtt ttctgtgtcc 8040
agccagagaa aggaggccgc aagccagctc gccttatcgt attcccagac ctgggagttc 8100
gtgtgtgcga gaagatggcc ctctacgatg tggtctccac tcttcctcag gccgtgatgg 8160
gctcctcata cgggttccag tactctccta aacagcgggt tgaattcctg gtgaacacct 8220
ggaaatcaaa gaaatgccct atgggcttct catatgacac ccgctgtttt gactcaacgg 8280
tcactgagaa tgacatccgt gttgaggagt caatttacca atgctgtgac ttggcccccg 8340
aagccaaact ggccataaag tcgctcacag agcggctcta tgttgggggt cccctgacta 8400
attcaaaagg gcagaactgc ggttatcgcc ggtgccgcgc gagcggcgtg ctgacgacta 8460
gctgcggtaa taccctcaca tgttatctga aagcctctgc ggcctgtcga gctgcaaagc 8520
tccaggactg cacgatgctc gtgaacggag acgaccttgt cgttatctgt gaaagcgcgg 8580
gaacccaaga ggatgcggcg agcctacgag tcttcacgga ggctatgact aggtattctg 8640
ccccccccgg ggacccgcct caaccagaat acgacttgga gttgataaca tcatgttcct 8700
ccaatgtgtc ggtcgcacac gatgcatctg gtaaaagggt gtactacctc acccgtgacc 8760
ccaccacccc ccttgcgcgg gctgcgtggg agacagctag acacactcca gtcaactcct 8820
ggctaggcaa tatcatcatg tatgcgccta ccttatgggc aaggatgatt ctgatgaccc 8880
acttcttctc catccttcta gctcaggagc agcttgaaaa agccctggat tgccagatct 8940
acggggcctg ttactccatt gagccacttg acctacctca gatcattgag cggctccatg 9000
gtcttagcgc attttcactc catagttact ctccaggtga gatcaatagg gtggcttcat 9060
gcctcaggaa acttggggtc ccacccttgc gagtctggag acatcgggcc agaagtgtcc 9120
gcgctaagtt actgtcccag ggggggaggg ccgccacttg tggcaagtac ctcttcaact 9180
gggcagtaaa gaccaagctt aaactcactc caattccggc tgcgtcccgg ttggacttgt 9240
ccggctggtt cgtcgccggt tacagcgggg gagacatata tcacagcctg tctcgcgccc 9300
gaccccgctg gttcatgtgg tgcctactcc tactttccgt aggggtaggc atctacctgc 9360
tccccaaccg gtgaacgggg agctaaacac tccaggccaa taggccattc cttttttttt 9420
tttttttttt tttttctttt tttttccttt tttttttttt tttttttttt ttcctttttct 9480
ttcttctttg gtggctccat cttagcccta gtcacggcta gctgtgaaag gtccgtgagc 9540
cgcatgactg cagagagtgc tgatactggc ctctctgcag atcatgt 9587